# EUROPÄISCHE PATENTSCHRIFT

(11) **EP 1 629 774 B1**
(45) Veröffentlichungstag und Bekanntmachung des Hinweises auf die Patenterteilung: **04.03.2009**
(21) Anmeldenummer: 04020197.2
(22) Anmeldetag: 25.08.2004
(51) Int. Cl.: A61B 5/06, A61B 19/00

(54) **Registrierung von intraoperativen Scans**
Registration of intraoperative scans
Enregistrement de balayages peropératoires

(43) Veröffentlichungstag der Anmeldung: 01.03.2006
(73) Patentinhaber: BrainLAB AG, 85622 Feldkirchen (DE)
(72) Erfinder: Bertram, Michael, 85570 Markt Schwaben (DE); Essenreiter, Robert, 81825 München (DE)
(74) Vertreter: Schwabe - Sandmair - Marx

(56) Entgegenhaltungen:
- EP-A- 1 378 206
- WO-A-01/01845
- WO-A-01/87136
- WO-A2-20/04023103

## Beschreibung

Die Erfindung betrifft eine Vorrichtung und ein Verfahren zum automatischen Registrieren von Bilddaten, insbesondere intraoperativen CT-Scans, zum Beispiel zur Verwendung bei einem Navigationsverfahren, um zum Beispiel die Auswirkungen der.Veränderung eines Körpers nach einem chirurgischen Eingriff erfassen zu können.

Wird durch einen chirurgischen Eingriff, zum Beispiel bei einer Becken- oder Gehirnoperation, durch das Eingreifen selbst, zum Beispiel durch das Vorschieben eines Führungsdrahtes, oder Folgeerscheinungen, wie zum Beispiel dem Absacken von Organen oder Gewebe, die interne Struktur eines Körpers verändert, so kann es häufig nützlich sein, dass intraoperativ, das heißt nach einem chirurgischen Eingriff und vor einem weiteren chirurgischen Eingriff, Bilddaten des Körpers erfasst werden müssen, um die durch den chirurgischen Eingriff hervorgerufenen Änderungen zu erfassen. So kann zum Beispiel bei einer Beckenoperation mittels eines oder mehrerer intraoperativer Scans überprüft werden, an welcher Position sich der Bohrer im Knochen befindet. Hierzu wird zum Beispiel ein Patient mit einem an ihm befestigten Marker in einen Computertomographen geschoben, um eine Computertomographie durchzuführen und anschließend wieder aus dem Computertomographen herausgeschoben, um einen weiteren chirurgischen Eingriff vornehmen zu können, wobei es vorteilhaft ist, wenn der Patient immobilisiert ist, also sich zum Beispiel in einer festen Position relativ zu einem Tisch befindet.

Die US 6,678,545 B2 offenbart ein System zur Bestimmung der Position einer Sonde innerhalb eines Kopfes, wobei ein entsprechendes Bild angezeigt wird.

Die WO 01/01845 A2 bezieht sich auf Systeme und Verfahren für medizinische Eingriffe mittels medizinischer bildgebender Systeme, wobei durch die Verwendung von Lokalisierungsvorrichtungen bildgestützte medizinische Eingriffe durchgeführt werden können, indem die Position eines medizinischen Instruments in Bezug auf ein von der medizinischen bildgebenden Vorrichtung erzeugtes Bild und/oder in Bezug auf den Patienten berechnet wird.

Die EP 1 378 206 A1 betrifft ein Verfahren zur Bilddarstellung bei einem medizinischen Navigationssystem mit Röntgenbildunterstützung, wobei zusätzlich zu aus erstellten, ausgewerteten und umgesetzten Röntgenbildern erhaltenen Patientendaten noch die ursprünglichen Röntgenbilder an ein Navigationssystem übergeben werden und die zweidimensionalen Röntgenbilder navigationsunterstützend auf einer Bildausgabe des Navigationssystems angezeigt werden.

Die WO 01/87136 A2 bezieht sich auf ein fluoroskopisches Tracking- und Visualisierungssystem, wobei Marker an den Bilddetektor des Fluoroskops und an einem Instrument und einem Patienten befestigt werden, um die jeweiligen Positionsdaten zu erhalten. Ein Markerdetektionsmodul identifiziert die abgebildeten Marker in jedem Foto und ein Prozessor errechnet aus den bekannten Markerpositionen zusammen mit den verfolgten Positionen des Instruments ein Instrumentennavigationsbild

Die WO 2004/023103 A2 beschreibt ein Verfahren, wobei ein Patient gescannt wird, um Scanner-Bilder zu erhalten, eine Position eines dem Patienten zugeordneten Patienten-Tracker bestimmt wird, eine Transformation zwischen dem Patienten-Tracker und dem Scanner berechnet wird und eine Transformation zwischen dem Patienten-Tracker und den Scanner-Bildern bestimmt wird, basierend auf der Transformation zwischen dem Patienten-Tracker und dem Scanner und dem bekannten Verhältnis des Scanners relativ zu den Scanner-Bildern.

Es ist eine Aufgabe der Erfindung ein Verfahren und eine Vorrichtung zum Registrieren von Bilddaten eines Körpers vorzuschlagen, welche den Schritt der Erfassung von Bilddaten verbessern können.

Diese Aufgabe wird durch die Gegenstände der unabhängigen Ansprüche gelöst. Vorteilhafte Ausführungsformen ergeben sich aus den abhängigen Ansprüchen.

Bei dem erfindungsgemäßen Verfahren zum Registrieren von Bilddaten eines Körpers werden die Bilddaten des Körpers in einem Erfassungsbereich, zum Beispiel in einem Kernspintomographen oder einem Computertomographen, erfasst, wobei es nicht erforderlich ist, dass während des Erfassungsvorganges ein Referenzstern oder andere Marker auf dem Körper des Patienten selbst oder bei dem Patienten zum Beispiel auf einem Tisch, auf welchem der Patient liegt, vorhanden sind. Das Erfassen der Bilddaten kann auf verschiedene, aus dem Stand der Technik bekannte Arten durchgeführt werden. Zum Beispiel kann der Körper oder der Tisch während des Aufnahmevorgangs mit einem konstanten kontinuierlichen Vorschub, wie bei einer Spiral-Computertomographie, durch den Erfassungsbereich geschoben werden, oder der Vorschub kann schrittweise erfolgen, indem jeweils nach einem Vorschub des Tisches um eine bestimmte Länge in einer unbewegten feststehenden Position des Tisches eine Aufnahme erfolgen kann bis der Körper oder der Tisch den Erfassungsbereich verlässt oder der gewünschte Bereich aufgezeichnet wurde. Zum anderen kann sich auch die Erfassungsvorrichtung, also der Erfassungsbereich entlang des Körpers bewegen, um den Körper oder Teile des Körpers schrittweise oder mit zum Beispiel konstantem Vorschub aufzunehmen, indem zum Beispiel die Datenerfassungsvorrichtung auf Schienen parallel zu dem Körper oder dem Tisch gefahren wird und der Tisch bzw. der Körper stillsteht. Unabhängig davon, ob der Körper und/oder der Erfassungsbereich bewegt wird, kann von einer Bewegung des Körpers relativ zu dem Erfassungsbereich gesprochen werden. Der Körper oder der Tisch kann somit relativ zum Erfassungsbereich verschoben werden oder umgekehrt, das heißt der Erfassungsbereich kann relativ zu dem Körper oder dem Tisch verschoben werden.

Der Körper kann nach dem Erfassen der Bilddaten aus dem Erfassungsbereich, also zum Beispiel aus dem Kernspintomographen, heraus verschoben werden oder der Erfassungsbereich kann relativ zu dem Körper verschoben werden, wobei zum Beispiel erst nach dem Verschieben des Körpers oder des Patienten, zum Beispiel nach dem Ausfahren eines Tisches zur Lagerung des Patienten, an dem Patienten selbst oder an dem Tisch, auf welchem der Patient bevorzugt unbeweglich gelagert ist, Marker, wie zum Beispiel ein erstes Markerarray, bestehend aus drei, vier oder mehr Marken oder ein Referenzstern befestigt werden können.

Sind Marker, wie zum Beispiel ein zweites Markerarray bestehend aus drei, vier oder mehr Markern oder auch ein Referenzstern, an einer definierten Position relativ zum Erfassungsbereich, also zum Beispiel an der Erfassungsvorrichtung, wie zum Beispiel dem Computertomographen selbst, befestigt, so kann der aus dem Erfassungsbereich herausgeschobene Körper registriert werden, wenn die Vorschiebungsrichtung und ―weite und eine eventuelle Drehung bekannt ist, also wenn zum Beispiel erfasst wird wie weit und in welche Richtung der Körper aus dem Erfassungsbereich herausgeschoben wurde.

Zunächst ist der in dem Erfassungsbereich liegende Körper registriert und könnte zum Beispiel durch das in einem definierten Lageverhältnis zum Erfassungsbereich, zum Beispiel am Scanner selbst, angeordnete zweite Markerarray, welches mittels eines ersten Transformations- oder Registrierungsverfahrens bestimmt werden kann, navigiert werden. Dieses erste Transformations- oder Registrierungsverfahren muss erst dann erneut durchgeführt werden, wenn der oder die relativ zu dem Erfassungsbereich angeordneten Marker versetzt, also zum Beispiel an einer anderen Stelle der Datenerfassungsvorrichtung, wie dem Kernspintomographen angebracht werden. Der Körper des Patienten ist also nach einer Aufnahme im Erfassungsbereich registriert, wenn die Marker bzw. das zweite Markerarray nicht versetzt wurden. Da der Körper oder der Tisch jedoch zum Beispiel für einen weiteren chirurgischen Eingriff aus dem Erfassungsbereich herausbewegt werden können, kann nach dem Herausbewegen des Körpers oder des Tisches aus dem Erfassungsbereich an dem Körper selbst oder in einem festen relativen Lageverhältnis zu dem Körper, wie zum Beispiel am Tisch, das erste Markerarray angebracht werden. Die genaue Position oder der Abstand des ersten Markerarrays relativ zu dem Körper muss nicht bekannt sein, das erste Markerarray kann jedoch unbeweglich angeordnet sein, so dass es einen konstanten Abstand oder ein festes Lageverhältnis oder eine feste Position relativ zu dem vorzugsweise unbeweglich gelagerten Körper besitzt. Der Körper kann dann registriert werden, wenn das Lageverhältnis zwischen dem ersten und dem zweiten Markerarray bekannt ist, was als ein zweites Transformationsverfahren bezeichnet wird. Hierzu kann zum Beispiel eine Information bezüglich der räumlichen Verschiebung des Körpers aus dem Erfassungsbereich heraus als ein drittes Transformationsverfahren verwendet werden. Ist das relative Lageverhältnis des zweiten Markerarrays zum Erfassungsbereich, also die erste Transformation, und die Translation oder Verschiebung des Körpers aus dem Erfassungsbereich heraus, also die dritte Transformation bekannt, so kann der Körper registriert werden. Dabei kann die zweite Transformation, d.h. das Lageverhältnis der ersten zur zweiten Markeranordnung ermittelt werden, um den Körper mit der ersten Markeranordnung navigieren zu können.

Das erste Markerarray kann auch an dem Körper und/oder dem Tisch, auf welchem der Körper vorzugsweise unbeweglich gelagert ist, mittels eines Referenzsternadapters wie in der EP 04 003 019 der Anmelderin beschrieben angebracht werden, wobei die Lehre der EP 04 003 019 bezüglich der Ausgestaltung eines Referenzsternadapters in diese Anmeldung aufgenommen wird. Ein solcher Referenzsternadapter ermöglicht, dass ein an einem Körper oder Instrument, wie dem Tisch, mittels des Referenzsternadapters befestigter Referenzstern automatisch oder manuell neu positioniert wird, also zum Beispiel um ein oder mehrere Gelenke gedreht und/oder entlang einer oder mehrerer vorgegebener Richtungen verschoben werden kann, wobei durch mindestens einen Positionsdetektor, der mit einem zwischen dem Referenzstern und dem Referenzsternadapter liegenden Lager verbunden ist, ermittelt werden kann, welche Bewegung der Referenzstern in Bezug auf seine bisherige Position ausgeführt hat. Somit ist eine neue Registrierung nicht erforderlich, da aus der durch den mindestens einen Positionsdetektor ermittelten Lageveränderung des Referenzsterns das neue Lageverhältnis zwischen dem Referenzstern und dem mit diesem Referenzstern verbundenen Körper oder Tisch bestimmt werden kann, womit der Referenzstern wieder in einem definierten Lageverhältnis zu diesem Körper oder diesem Tisch steht.

Die dritte Transformation kann zum Beispiel durch das Lageverhältnis des aus dem Erfassungsbereich verschobenen Körpers relativ zu dem in dem Erfassungsbereich liegenden Körpers beschrieben werden, wobei zum Beispiel die Verschiebung des Tisches, auf welchem der Körper liegt, ermittelt wird. Dabei kann zum Beispiel die Position des Tisches oder des Körpers nach dem Herausschieben mittels eines Detektors erfasst werden oder der Tisch oder Körper kann an eine bekannte Position herausgeschoben werden.

Mit Hilfe des zweiten Transformationsverfahrens, können die erfassten Bilddaten, welche bezüglich eines Koordinatensystems, dessen Ursprung zum Beispiel durch das zweite Markerarray gebildet wird, definiert sind, in ein anderes Koordinatensystem transformiert werden, dessen Ursprung zum Beispiel durch das relativ zu dem Körper in einem festen Lageverhältnis angeordnete erste Markerarray gebildet wird. Damit ist die räumliche Lage der Bilddaten relativ zu dem ersten Markerarray bekannt, so dass sie an jedem Ort eindeutig bezüglich des ersten Markerarrays bestimmt sind und zum Beispiel bei einer Operation eindeutig einer bestimmten Stelle oder einem bestimmten Bereich des Körpers eines Patienten zugeordnet werden können. Solange sich der Körper in dem Erfassungsbereich befindet, sind die Bilddaten durch die erste Transformation definiert, womit bekannt ist, an welcher Stelle des Körpers die jeweiligen Daten aufgenommen wurden. Nach Herausschieben des Körpers können, zum Beispiel durch Ermittlung wie weit der Tisch oder der Patient herausgeschoben wurde, die Bilddaten bezüglich des ersten Markerarrays zum Beispiel mit Hilfe der zweiten Transformation definiert werden, welches zum Beispiel an dem Tisch angebracht wird oder ist.

Die Erfassung der Bilddaten ist nicht auf ein bestimmtes bildgebendes Verfahren beschränkt, sondern kann durch ein beliebiges Verfahren, wie zum Beispiel ein Computertomographie-, ein Kernspinresonanz-, ein Positronen-Emissions-Tomographie (PET)-, ein SPECT (Single Photon Emission Computed To-mography)- oder ein Ultraschallverfahren durchgeführt werden.

Das erfindungsgemäße Verfahren zur Registrierung von Bilddaten kann insbesondere auch als ein Computerprogramm gestaltet sein. Dieses Computerprogramm kann, wenn es in einen Computer geladen ist oder auf einem Computer läuft, das erfindungsgemäße Verfahren insbesondere automatisiert ausführen, so dass kein Eingreifen durch einen Anwender nötig ist. So könnten die Marker zum Beispiel mittels eines Steuerprogramms automatisch gesetzt werden, der Tisch automatisch in eine bestimmte Position aus dem Erfassungsbereich heraus verschoben werden, oder die Transformations- oder Registrierungsverfahren automatisch durchgeführt werden. Das Computerprogramm kann zum Beispiel in einem Programmspeichermedium oder einem Computerprogrammprodukt enthalten sein.

Die Vorrichtung zum Registrieren von Bilddaten eines Körpers umfasst eine bevorzugt beweglich ausgebildete Kamera, insbesondere eine Infrarotkamera, ein erstes Markerarray, insbesondere ein Infrarot-Strahlung reflektierendes oder emittierendes Markerarray, welches in einem festen Lageverhältnis relativ zum Körper des Patienten angeordnet ist, sowie ein zweites Markerarray, insbesondere ein Infrarot-Strahlung reflektierendes oder emittierendes Markerarray, welches an einer bekannten festen, bezüglich der räumlichen Lage definierten Position angeordnet ist. Dabei ist das erste Markerarray bevorzugt unbeweglich in einem konstanten Lageverhältnis oder Abstand relativ zu dem Körper, zum Beispiel an dem Körper selbst oder an einer Position mit konstanter Entfernung relativ zu dem Körper, angeordnet. Ist die Lage der Markerarrays zueinander bekannt, ist auf Grund der bekannten Position des zweiten Markerarrays, insbesondere auf Grund des bekannten Lageverhältnisses des zweiten Markerarrays relativ zum Erfassungsbereich, auch die Lage des ersten Markerarrays zum aus dem Erfassungsbereich verschobenen Körper bekannt, wobei die Position des zweiten Markerarrays für die weitere Navigation nicht von Bedeutung ist.

Bevorzugt umfasst die Vorrichtung eine Datenerfassungsvorrichtung zum Erfassen von Bilddaten eines Körpers, wie einen Computertomographen, einen Kernspin- bzw. Magnetresonanztomographen oder ein Ultraschallgerät, wobei das zweite Markerarray zum Beispiel an der Datenerfassungsvorrichtung befestigt ist und damit eine relativ zum Erfassungsbereich bekannte Position hat und folglich dazu ge-nutzt werden kann, den Körper des Patienten nach dem des Aufnahme- bzw. Scan-Vorgang zu registrieren.

Vorzugsweise umfasst die Vorrichtung zum Registrieren von Bilddaten eines Körpers einen Tisch, auf welchem sich der Körper eines Patienten befinden kann und welcher insbesondere in den Erfassungsbereich hinein und aus dem Erfassungsbereich heraus bewegt werden kann. Insbesondere kann auch der Erfassungsbereich entlang des Körpers bewegt werden, indem zum Beispiel die Datenerfassungsvorrichtung auf zum Beispiel parallel zu dem Tisch oder Körper verlaufenden Schienen bewegt wird und der Tisch oder der Körper stillsteht und/oder bewegt wird. Dabei kann das erste Markerarray an dem Tisch zum Beispiel in einem festen Lageverhältnis relativ zum Körper des Patienten befestigt sein.

Das erste Markerarray besitzt somit relativ zu dem Körper des Patienten, der bevorzugt unbeweglich gelagert ist, stets die selbe Position, egal ob sich der Tisch in dem Erfassungsbereich des Datenerfassungsgeräts, in einem Operationssaal oder an einem anderen Ort befindet. Die Lage des zweiten Markerarrays relativ zu dem Erfassungsbereich ist durch das zum Beispiel einmal vor der Aufnahme bestimmte erste Transformationsverfahren oder zum Beispiel mit einem Phantom mit Markern durchgeführte Registrierungsverfahren bekannt. Die Lage des Körpers und damit die Lage der aufgenommenen Bilddaten während des Aufnahmevorgangs relativ zu der Lage des Körpers und damit der Lage der erfassten Bilddaten nach Herausschieben des Tisches aus dem Erfassungsbereich sind durch das dritte Transformations- oder Registrierungsverfahren bekannt. Mit Hilfe dieser Lagebeziehungen und der Lage der beiden Markerarrays zueinander kann der aus dem Erfassungsbereich herausgeschobene Körper registriert werden.

Die Erfindung wird nachfolgend anhand bevorzugter Ausführungsbeispiele unter Bezugnahme auf die Zeichnungen beschrieben werden. Es zeigen:
- Figur 1: eine Ausführungsform einer erfindungsgemäßen Vorrichtung zum Erfassen von Bilddaten eines Körpers während eines ScanVorganges; und
- Figur 2: eine Ausführungsform einer erfindungsgemäßen Vorrichtung zum Registrieren von Bilddaten eines Körpers nach dem Scannen während eines Registrierungs-Vorganges.

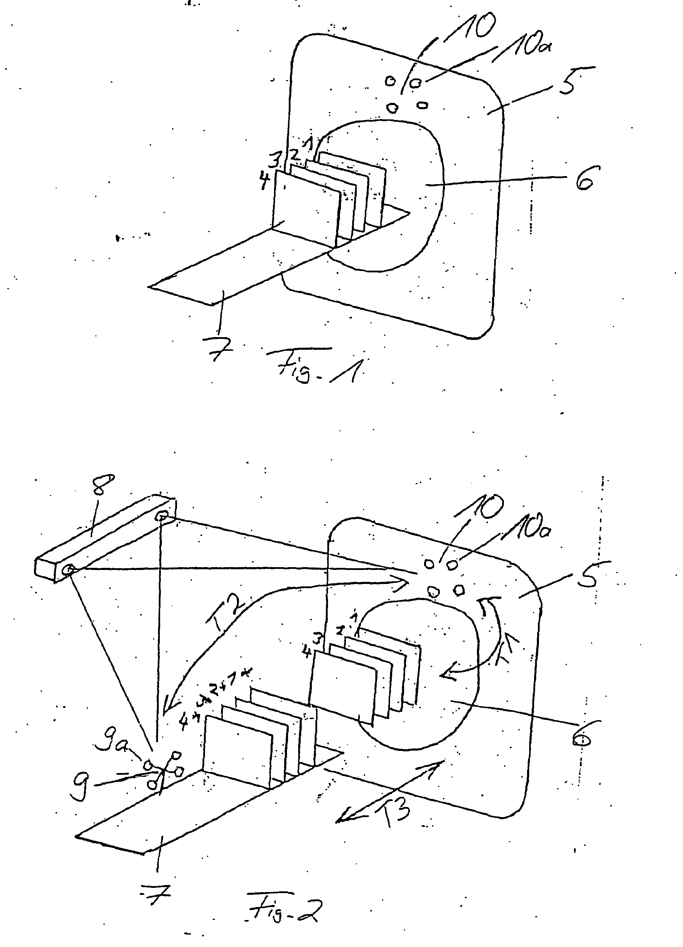

Figur 1 zeigt eine Vorrichtung zum Erfassen von Bilddaten eines Körpers, wobei die Datenerfassungsvorrichtung 5 als ein Computertomograph ausgebildet ist, auf welchem an einer bestimmten Position, die während des Scan-Vorganges sowie während des nachfolgenden Registrierungs-Vorganges nicht verändert wird, ein zweites Markerarray 10 bestehend aus vier Markern 10a befestigt ist. Auf dem vorzugsweise beweglichen Tisch 7 des Computertomographen befindet sich der Körper eines Patienten, wobei die durch die Aufnahme im Erfassungsbereich 6 erfassten Bilddaten, insbesondere bestimmte aufgenommene Schichten des Körpers, durch die Ebenen 1 bis 4 veranschaulicht werden. Diese Schichten bzw. Aufnahmen 1 bis 4 können, wie aus dem Stand der Technik bekannt, ermittelt bzw. erfasst werden, wobei während des Aufnahmeverfahrens der Tisch 7 zum Beispiel mit kontinuierlichem Vorschub bewegt werden kann, wie bei einer Spiral-Computertomographie, oder schrittweise, mit während der Aufnahme feststehendem Tisch 7, durch den unbewegten Erfassungsbereich 6 bewegt werden kann. Auch könnte der Tisch 7 während des gesamten Aufnahmeverfahrens nicht bewegt werden und die Datenerfassungsvorrichtung 5 mit deren Erfassungsbereich 6 kontinuierlich oder schrittweise entlang des Tisches 7 bewegt werden.

In Figur 2 ist die erfindungsgemäße Vorrichtung während eines Registrierungs-Vorganges nach dem Scannen und Herausschieben des Tisches veranschaulicht, wobei die mittels des Scan-Vorganges erfassten Bilddaten im Erfassungsbereich 6 durch die Schichten 1 bis 4 und die zugehörigen Bereiche des herausgeschobenen Körpers 1* bis 4* verdeutlicht werden. Bei einem ersten Transformations- oder Registrierungsverfahren T1 wurde die relative Lage des Erfassungsbereichs 6, also zum Beispiel der aufgenommenen Bilddaten, zu dem an dem Computertomographen befestigten zweiten Markerarray 10 bestimmt, womit die Schichten 1 bis 4 der aufgenommenen Bilddaten eindeutig den jeweiligen Stellen des aufgenommenen Körpers im Erfassungsbereich 6 zugeordnet werden können, solange sich der Patient in dem Computertomographen befindet. Diese exakte Zuordnung ist nach dem Herausschieben des Patienten aus dem Erfassungsbereich 6 des Computertomographen zunächst nicht mehr möglich. Wird nun, wie in Figur 2 dargestellt, ein weiteres Markerarray 9 zum Beispiel in Form eines Referenzsternes oder bestehend aus vier Markern auf dem Tisch 7 aufgebracht und wird mit einem weiteren Transformationsverfahren T3 beschrieben wie weit der Körper des Patienten aus dem Erfassungsbereich 6 herausgeschoben wurde, oder ist bekannt wie weit der Körper des Patienten aus dem Erfassungsbereich 6 zum Beispiel an eine definierte Position herausgeschoben wurde, so kann aus der ersten Transformation T1 und der dritten Transformation T3 unter Kenntnis des Lageverhältnis der Markerarrays zueinander durch die Transformation T2 der Körper registriert werden, d.h. die Aufnahmedaten 1 bis 4 können den Bereichen des Körpers 1* bis 4* zugeordnet werden, wenn bei der nachfolgenden Navigation nur noch die Lage des ersten Markerarrays 9 erfasst wird, d.h., die Kamera so bewegt wird, dass das zweite Markerarray 10 für die Kamera nicht mehr sichtbar ist. Damit können die erfassten Schichten 1 bis 4 des Körpers so zugeordnet werden, dass deren Lage bezüglich des auf dem Tisch angeordneten ersten Markerarrays 9 bekannt ist, siehe Schichten 1* bis 4*, womit eine eindeutige Zuordnung der aufgenommenen Bilddaten an die zugehörige Stelle des Körpers des Patienten möglich ist, unabhängig davon wo sich der Patient befindet bzw. wohin der Tisch 7 verschoben wurde. Da das erste Markerarray 9 erst nach dem Aufnahmevorgang des Computertomographen 5 angebracht werden kann, kann eine Störung, zum Beispiel durch das an dem Tisch 7 befestigte erste Markerarray 9, während des Scan-Vorgangs oder beim Einschieben des Patienten bzw. des Tisches 7 in den Erfassungsbereich 6, verhindert werden.

## Patentansprüche

1. Verfahren zum Registrieren von Bilddaten eines Körpers, wobei die Bilddaten des Körpers in einem Erfassungsbereich (6) erfasst werden, der Körper relativ zum Erfassungsbereich (6) verschoben wird oder umgekehrt und aus der erfassten Position eines relativ zu dem Körper in einem festen Lageverhältnis angeordneten ersten Markerarrays (9) und aus der erfassten Position eines relativ zu dem Erfassungsbereich (6) in einem definierten Lageverhältnis angeordneten zweiten Markerarrays (10) die Registrierung der Bilddaten durchgeführt wird, **dadurch gekennzeichnet, dass** das erste Markerarray (9) zur Registrierung des Körpers erst nach dem Erfassen der Bilddaten des Körpers in dem festen Lageverhältnis relativ zum Körper angebracht wird.

2. Verfahren nach Anspruch 1, wobei das erste Markerarray (9) zum Registrieren des Körpers nach dem Verschieben des Körpers aus dem Erfassungsbereich (6) in einem festen Lageverhältnis relativ zum Körper angebracht wird.

3. Verfahren nach einem der vorhergehenden Ansprüche, wobei Daten zur Beschreibung einer dritten Transformation ermittelt werden, welche das Lageverhältnis des aus dem Erfassungsbereich (6) verschobenen Körpers relativ zu dem in dem Erfassungsbereich (6) liegenden Körpers beschreibt.

4. Verfahren nach einem der vorhergehenden Ansprüche, wobei ein erstes Transformations- oder Registrierungsverfahren durchgeführt wird, um das Lageverhältnis des zweiten Markerarrays (10) relativ zum Erfassungsbereich (6) zu definieren.

5. Verfahren nach Anspruch 3, wobei die Daten zur Beschreibung der dritten Transformation aus der Verschiebung eines Tisches (7), auf welchem der Körper liegt, aus dem Erfassungsbereich (6) heraus ermittelt werden.

6. Verfahren nach einem der drei vorhergehenden Ansprüche, wobei Daten für eine zweite Transformation, mit welcher die Bilddaten des Körpers registriert werden können, aus den Daten der ersten und der dritten Transformation ermittelt werden.

7. Verfahren nach einem der vorhergehenden Ansprüche, wobei die Bilddaten des Körpers durch ein Computertomographie-, ein Kernspinresonanz-, ein Positronen-Emissions-Tomographie-, ein SPECT (Single Photon Emission Computed Tomography)- oder ein Ultraschallverfahren erfasst werden.

8. Computerprogramm, welches, wenn es in einem Computer geladen ist oder auf einem Computer läuft, das Verfahren nach einem der vorhergehenden Ansprüche ausführt.

9. Programmspeichermedium oder Computerprogrammprodukt mit dem Computerprogramm nach dem vorhergehenden Anspruch.

10. Vorrichtung zum Registrieren von Bilddaten eines Körpers mit einer Kamera (8) und einem ersten Markerarray (9), das in einem festen Lageverhältnis relativ zum Körper angeordnet werden kann, wobei ein zweites Markerarray (10) an einer bekannten festen Position angeordnet ist, mit einer Datenerfassungsvorrichtung (5) zum Erfassen von Bilddaten eines Körpers, insbesondere einem Computertomographen, einem Kernspintomographen oder einem Ultraschallgerät, wobei das zweite Markerarray (10) an der Datenerfassungsvorrichtung (5) befestigt ist, mit einem Tisch (7), welcher in den Erfassungsbereich (6) hinein und aus dem Erfassungsbereich (6) heraus bewegt werden kann oder umgekehrt, und mit einer Zuordnungseinheit, welche die Lage der erfassten Bilddaten relativ zu dem zweiten Markerarray (10) bestimmen kann, und unter Berücksichtigung, wie weit der Körper aus dem Erfassungsbereich heraus geschoben wurde, und unter Berücksichtigung des Lageverhältnisses des ersten und des zweiten Markerarrays (9, 10) zueinander, die Bilddaten so dem ersten Markerarray (9) zuordnen kann, dass die Lage der Bilddaten bezüglich des ersten Markerarrays (9) definiert wird, wobei das erste Markerarray (9) zur Registrierung des Körpers erst nach dem Erfassen der Bilddaten des Körpers in dem festen Lageverhältnis relativ zum Körper angebracht wird.

11. Vorrichtung nach dem vorhergehenden Anspruch, wobei die Kamera (8) beweglich ausgebildet ist.

12. Vorrichtung nach einem der beiden vorhergehenden Ansprüche, wobei das erste Markerarray (9) an dem Tisch (7) befestigt ist.

## Claims

1. A method for registering image data of a body, wherein the image data of the body are captured in a detection range (6), the body is shifted relative to the detection range (6) or vice versa, and the image data are registered from the detected position of a first marker array (9), arranged in a fixed positional relationship relative to the body, and from the detected position of a second marker array (10), arranged in a defined positional relationship relative to the detection range (6), **characterised in that** the first marker array (9) for registering the body is not attached in the fixed positional relationship relative to the body until after the image data of the body have been captured.

2. The method according to claim 1, wherein the first marker array (9) for registering the body is attached in a fixed positional relationship relative to the body, once the body has been shifted out of the detection range (6).

3. The method according to any one of the preceding claims, wherein data for describing a third transformation are ascertained, which describes the positional relationship of the body removed from the detection range (6) relative to the body lying in the detection range (6).

4. The method according to any one of the preceding claims, wherein a first transformation or registration method is performed in order to define the positional relationship of the second marker array (10) relative to the detection range (6).

5. The method according to claim 3, wherein the data for describing the third transformation are ascertained from the shift of a table (7), on which the body is lying, out of the detection range (6).

6. The method according to any one of the preceding three claims, wherein data for a second transformation, using which the image data of the body can be registered, are ascertained from the data of the first and third transformation.

7. The method according to any one of the preceding claims, wherein the image data of the body are captured using a computer tomography method, a nuclear spin resonance method, a positron emission tomography method, a SPECT (single photon emission computed tomography) method or an ultrasound method.

8. A computer program which, when it is loaded onto a computer or is running on a computer, performs the method according to any one of the preceding claims.

9. A program storage medium or computer program product comprising the computer program according to the preceding claim.

10. A device for registering image data of a body, comprising a camera (8) and a first marker array (9) which can be arranged in a fixed positional relationship relative to the body, wherein a second marker array (10) is arranged at a known fixed position, comprising: a data capture device (5) for capturing image data of a body, in particular a computer tomograph, a nuclear spin tomograph or an ultrasound apparatus, wherein the second marker array (10) is attached to the data capture device (5); a table (7) which can be moved into and out of the detection range (6) or vice versa; and an assigning unit which can determine the position of the captured image data relative to the second marker array (10), and by taking into account how far the body has been removed from the detection range and by taking into account the positional relationship of the first and second marker array (9, 10) relative to each other, can assign the image data to the first marker array (9), such that the position of the image data with respect to the first marker array (9) is defined, wherein the first marker array (9) for registering the body is not attached in the fixed positional relationship relative to the body until after the image data of the body have been captured.

11. The device according to the preceding claim, wherein the camera (8) is formed to be movable.

12. The device according to any one of the preceding two claims, wherein the first marker array (9) is attached to the table (7).

## Revendications

1. Procédé pour l'enregistrement de données d'image d'un corps, les données d'image du corps étant acquises dans une zone d'acquisition (6), le corps étant déplacé par rapport à la zone d'acquisition (6) ou inversement et l'enregistrement des données d'image étant mis en oeuvre à partir de la position acquise d'une première série de marqueurs (9) disposée selon un rapport positionnel fixe par rapport au corps et à partir de la position acquise d'une seconde série de marqueurs (10) disposée selon un rapport positionnel défini par rapport à la zone d'acquisition (6), **caractérisé en ce que** la première série de marqueurs (9) destinée à l'enregistrement du corps n'est mise en place selon le rapport positionnel fixe par rapport au corps qu'après l'acquisition des données d'image du corps.

2. Procédé selon la revendication 1, dans lequel la première série de marqueurs (9) destinée à l'enregistrement du corps est mise en place selon dans un rapport positionnel fixe par rapport au corps après le déplacement du corps hors de la zone d'acquisition (6).

3. Procédé selon l'une quelconque des revendications précédentes, dans lequel des données destinées à la description d'une troisième transformation sont déterminées, qui décrit le rapport positionnel du corps déplacé hors de la zone d'acquisition (6) par rapport au corps se trouvant dans le secteur de collecte (6).

4. Procédé selon l'une quelconque des revendications précédentes, dans lequel un premier procédé de transformation ou d'enregistrement est mis en oeuvre pour définir le rapport positionnel de la seconde série de marqueurs (10) par rapport à la zone d'acquisition (6).

5. Procédé selon la revendication 3, dans lequel les données destinées à la description de la troisième transformation sont déterminées à partir du déplacement d'une table (7), sur laquelle le corps repose, hors de la zone d'acquisition (6).

6. Procédé selon l'une quelconque des trois revendications précédentes, dans lequel des données pour une deuxième transformation, avec laquelle les données d'image du corps peuvent être enregistrées, sont déterminées à partir des données des première et troisième transformations.

7. Procédé selon l'une quelconque des revendications précédentes, dans lequel les données d'image du corps sont acquises par un procédé de tomographie assistée par ordinateur, un procédé de résonance magnétique nucléaire, un procédé de tomographie par émission de positrons, un procédé SPECT (tomographie d'émission monophotonique = TEMP) ou un procédé à ultrasons.

8. Logiciel informatique, qui, lorsqu'il est chargé ou fonctionne dans un ordinateur, met en oeuvre le procédé selon l'une quelconque des revendications précédentes.

9. Support d'enregistrement logiciel ou produit logiciel informatique comprenant le logiciel informatique selon la revendication précédente.

10. Dispositif pour l'enregistrement de données d'image d'un corps avec une caméra (8) et une première série de marqueurs (9), qui peut être agencée selon un rapport positionnel fixe par rapport au corps, une seconde série de marqueurs (10) étant agencée dans une position fixe connue, avec un dispositif d'acquisition de données (5) pour l'acquisition de données d'image d'un corps, en particulier un tomographe assisté par ordinateur, un tomographe par résonance magnétique nucléaire ou un instrument à ultrasons, la seconde série de marqueurs (10) étant fixée au dispositif d'acquisition de données (5), avec une table (7), qui peut être déplacée vers l'intérieur de la zone d'acquisition (6) et vers l'extérieur de la zone d'acquisition (6) ou inversement, et avec une unité d'affectation, qui peut déterminer la position des données d'image acquises par rapport à la seconde série de marqueurs (10), et peut, en prenant en compte à quel point le corps a été poussé vers l'extérieur de la zone d'acquisition et en prenant en compte le rapport positionnel des première et seconde séries de marqueurs (9, 10) l'une par rapport à l'autre, affecter des données d'image à la première série de marqueurs (9) de sorte que la position des données d'image par rapport à la première série de marqueurs (9) est définie, la première série de marqueurs (9) destinée à l'enregistrement du corps n'étant mise en place selon le rapport positionnel fixe par rapport au corps qu'après l'acquisition des données d'image du corps.

11. Dispositif selon la revendication précédente, dans lequel la caméra (8) est réalisée de manière mobile.

12. Dispositif selon l'une des deux revendications précédentes, dans lequel la première série de marqueurs (9) est fixée à la table (7).
